(19) Europäisches Patentamt

European Patent Office

Office européen des brevets

(11) Publication number: **0 449 867 B1**

# EUROPEAN PATENT SPECIFICATION

(45) Date of publication of patent specification: **20.04.94**  (51) Int. Cl.⁵: **A61B 17/58**

(21) Application number: **90900201.6**

(22) Date of filing: **20.12.89**

(86) International application number:
**PCT/FI89/00236**

(87) International publication number:
**WO 90/07304 (12.07.90 90/16)**

(54) **POLYMERIC FIXATION PLATE FOR SURGICAL USE.**

(30) Priority: **23.12.88 FI 885981**

(43) Date of publication of application:
**09.10.91 Bulletin 91/41**

(45) Publication of the grant of the patent:
**20.04.94 Bulletin 94/16**

(84) Designated Contracting States:
**DE FR GB IT**

(56) References cited:
**EP-A- 0 149 540**
**WO-A-88/05312**
**US-A- 4 338 926**

(73) Proprietor: **BIOCON OY**
**Runeberginkatu 3 A 1**
**SF-33710 Tampere(FI)**

(72) Inventor: **POHJONEN, Timo**
**Nosturinraitti 2 B 17**
**SF-33720 Tampere(FI)**
Inventor: **TÖRMÄLÄ, Pertti**
**Runeberginkatu 3 A**
**SF-33710 Tampere(FI)**
Inventor: **VAINIONPÄÄ, Seppo**
**Orapihlajatie 21-27 B 12**
**SF-00320 Helsinki(FI)**
Inventor: **TAMMINMÄKI, Markku**
**Kukkolankatu 23 B 12**
**SF-33400 Tampere(FI)**

(74) Representative: **Smulders, Theodorus A.H.J.,**
**Ir. et al**
**Vereenigde Octrooibureaux**
**Nieuwe Parklaan 97**
**NL-2587 BN 's-Gravenhage (NL)**

# EP 0 449 867 B1

## Description

This invention describes a fixation device, which has been manufactured of a polymer, polymer mixture, copolymer or polymer composite and which is meant for fixation of bone fractures, osteotomies or arthrodeses. The device has been defined in more detail in the preamble of claim 1.

In orthopaedics and traumatology it is well known to use metallic plates for fixation of bone fractures, osteotomies or arthrodeses, in such a way that the plate bridges fracture-, osteotomy- or arthrodesis line and the plate is fixed into the bone with screws, bolts, rivets, rods, clamps or other corresponding fixation devices (see e.g. J. Schatzker and M. Tile "The Rationale of Operative Fracture Care", Springer, Berlin, 1987).

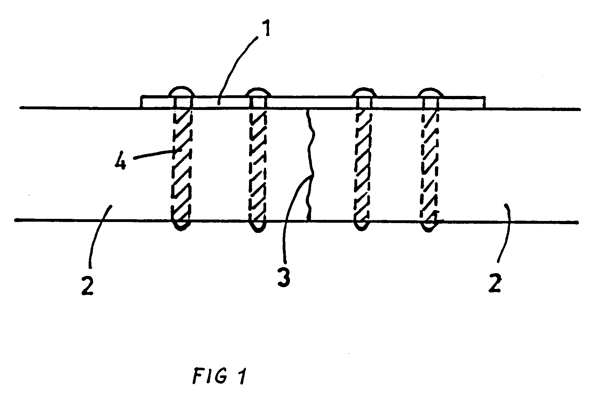

Figure 1 shows schematically as a side view in cross-section how metallic plate 1 immobilises the fracture 3 in a bone 2 when the plate has been fixed into bone on both sides of the fracture with fixation devices like screws 4. Plate - screw combination maintaines the contact between the bone parts and makes healing possible.

Metallic plates have, however, some negative properties. Metals are biomechanically too stiff materials (elastic modulus typically 100-200 GPa) in comparison to bone (elastic modulus typically 1-20 GPa). Therefore the metallic plate fixation protects bone too effectively against outer stresses. This leads to a so-called primary healing, which is slower than the healing with so-called callus mechanism, which happens with plates more flexible than the traditional metallic plates (see e.g. S.L.-Y. Woo, W.H. Akeson and R.D. Coutss in Perspectives on Biomaterials", O.C.C. Lind and E.Y.S. Chao (eds.), Elsevier, Amsterdam, 1986, pp. 223-244). Additionally the stiff metallic plate can lead to the increse of bone porosity.

Metallic corrosion can also often cause pains to patients. Therefore, after healing, the metallic plates must be removed in a second operation. After that the refracture of porous bone is possible.

An advantage of metallic plates is that they can easily be bend according to the form of the bone surface, which facilitates the creation of exact reduction. The bending can be done in an operation room with specific instruments. This is possible because fixation metals have strong plastic deformation capacity.

Many researchers have tried to develop polymeric fixation plates, especially of absorbable polymers and of polymer composites to eliminate the disadvantages of the metallic plates (the too high modulus, the increase of bone porosity, the metallic corrosion and the need of removal operation). In literature there are known for example polylactide plates (see e.g. L. Getter et al. "A biodegradable intraosseous appliance in the treatment of mandibular fractures", J. Oral. Surg. 30 (1972) 344-348), lactide plates reinforced with ceramic fibers (see e.g. D. Lewis et al. "Absorbable fixation plates with fiber reinforcement", 7th Ann. Meeting Biomater., Troy N.Y., 1981, p. 61) and lactide plates reinforced with carbon fibers (see e.g. H. Alexander et al. "Internal fracture fixation with partially degradable plates", Bioengineering - 9th Conf. Elmsford, New York, 1981, pp. 115-118).

EP-A-0 149 540 discloses a fracture fixation device comprising at least two laminae being bonded together such that repeated loading results in progressive failure of the interlaminar bond.

The most important weakness of known polymeric or polymer composite fixation plates is their poor plastic deformation in bending. It is possible to bend the plates but the deformation of polymers and polymer composites is at or near the room temperature mainly elastic and viscoelastic deformation, which is at least partially reversible. Therefore it is not possible to bend the plates at operation room temperature in a controlled way into a permanent form following the features of the bone surface. Only when the polymer or polymer composite plate is heated to a temperature $T > T_g$, where $T_g$ is the glass transition temperature of the polymer, it is possible to create plate the necessary strong plastic deformation into the plate (see e.g. J. Eitenmüller et al. "Treatment of ankle fractures with complete biodegradable plates and screws of high molecular weight polylactide", Trans. 3rd World Biomater. Congr., Kyoto, Japan, 1988, p. 195). The heating demands, however, special heating devices in the operation room. The plate must be heated, then bending as hot and it must be cooled before fixation on the bone. This makes the operation slow and complicated.

In this invention it has been found unexpectedly that when one uses a plate in accordance with the characteristic part of claim 1 instead one stiff polymeric and polymer composite plate at least two flexible plates, which essentially one on the other form a plate-like fixation device (multilayer plate) and which plates have such a thickness that they can be bend by hand and/or by means of a suitable bending instrument at room temperature, it is possible to eliminate the problems which are typical for the known polymeric or polymer composite fixation plates.

It is typical for the multilayer plate of the invention that it can before fixation easily be bent by hand or by a bending instrument, because the single plates of the multilayer plate glide in respect to each other.

2

Therefore it is possible to bend such multilayer plate at room or body temperature in operation room conditions on the surface of the uncovered fractured bone to follow exactly the features of the bone. Thereafter the multilayer plate can be fixed on the bone surface in the bended form temporarily e.g. by means of metallic clamps. Thereafter the bended multilayer plate can be locked into this desired form by drilling through the multilayer plate and the bone below it a suitable amount of drill holes and by fixing the bended multilayer plate into bone with screws, bolts, rivets, clamps etc. When the multilayer plate has been fixed tightly on the bone surface, it retains its bended form. The single plates of the multilayer plate can no more bend nor glide in respect to each other because of the compression friction between plates and fixation devices, like screws, which have been applied into the drill holes. In this case the multilayer plate, which is constructed of thin, flexible plates, functions like a thick and stiff bended plate giving an efficient fixation for the bone fracture.

The invention will be hereafter described in more detail with simultaneous references to the accompanied drawings, in which

Fig. 2a-d     shows schematically the steps of the fixation of the plate according to first embodiment of the invention,

Fig. 3a-d     shows schematically an alternative way of the steps of the fixation of the plate according to first embodiment of the invention,

Fig. 4a-d     shows schematically the second embodiment of the invention with oblong holes,

Fig. 5a-d     shows schematically yet another alternative way of the steps of the fixation of the plate according to first embodiment of the invention,

Fig. 6     shows schematically in a perspective view the different orientation of reinforcement elements in separate plates according to one alternative embodiment of the invention and

Fig. 7     shows a cross-sectional view of the embodiment of the invention used in connection with experiments of Example 2.

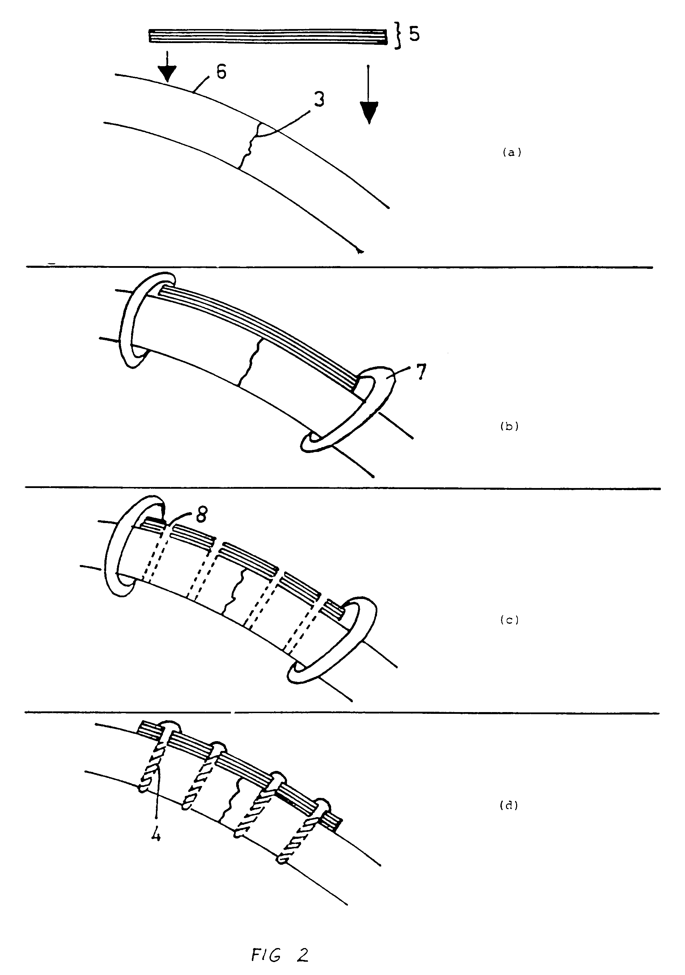

Figure 2 shows a side view in cross-section of a typical embodiment of the invention. A bundle 5 of four relatively thin, flexible plates is bent on the uncovered bone surface 6 over the fracture 3 (Figure 2a) and the bundle of the plates is fixed temporarily on the bone surface with metallic clamps 7 (Figure 2b). A suitable amount of holes 8 is drilled on the both sides of the fracture through the multilayer plate (or plate bundle) and through bone (Figure 2c). The multilayer plate 5 is fixed to the bone by means of screws 4 which are driven into the drill holes 8. The screws are typically driven through the back cortex to obtain strong fixation. The temporary clamps are removed (Figure 2d) and the uncovered bone surface can be covered with soft tissues. The reduction of the fracture is retained exact, because the single plates of the multilayer plate cannot glide in relation to each other.

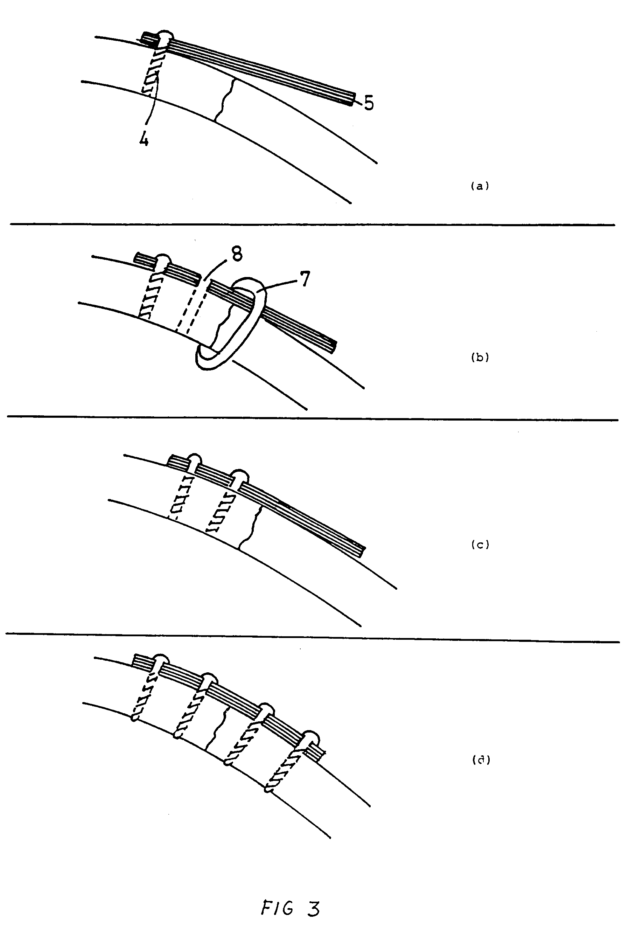

According to another advantageous embodiment (Figure 3) the multilayer plate 5 can be first fixed with one screw 4, with bolt, rivet, clamp or corresponding, to the surface of the uncovered bone through one drill hole (Figure 3a). Thereafter the multilayer plate can be fixed stepwise on the bone surface by bending the multilayer plate on the bone surface by fixing it temporarily with a metallic clamp 7, by drilling another hole 8 for the second screw through the multilayer plate and bone (Figure 3b) and by tightening the multilayer plate also through this hole on the bone by a screw. Now the clamp can be loosened (Figure 3c). The surgeon proceeds over the whole multilayer plate with this technique and as a consequence the multilayer plate will be fixed tightly on the bone (Figure 3d). It is self-evident that other versions of fixation techniques are possible for fixation of the multilayer plate of the invention on the bone.

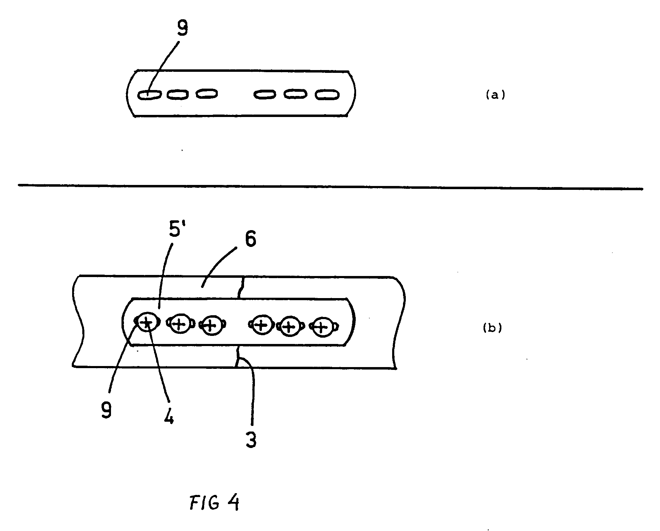

According to an advantageous embodiment the plates of the multilayer plate can have elongated holes. Figure 4a shows schematically a plan view from above one such plate of the multilayer plate with its elongated holes 9. When a bundle of this kind of plates is stacked on the bone surface, the fixation holes can be drilled directly into the bone through the elongated holes and the multilayer plate can be fixed with screws or other fixation elements on the bone. After tightening of the screws the plates cannot glide any more in relation to each other. This situation has been described schematically from above in Figure 4b, where the topmost plate 5' of the multilayer plate bridging the fracture 3 of the bone 6. The fixation screws 4 going through the elongated holes 9 are also seen.

With the multilayer plate of the invention one can create also compression into the bone fracture according to the following principles:

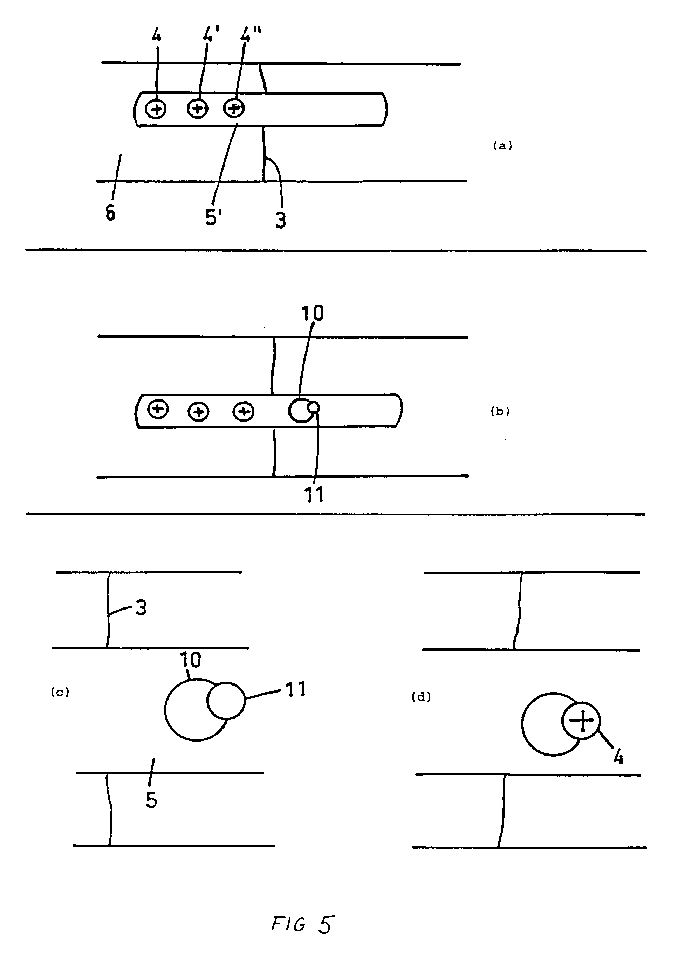

The multilayer plate is fixed with metallic clamps on the bone surface over the fracture in such a way that a good mutual contact between bone fragments, and between multilayer plate and bone is retained. The multilayer plate is fixed on bone first with one fixation device (e.g. with a screw according to Figure 3a). On the same side of the fracture additionally necessary amounts of screws is fixed (e.g. 2-3 additional screws). Figure 5a shows schematically from above the situation, where the multilayer plate 5 (whose uppermost plate 5' is seen) has been fixed on the bone 6 on the other side of the fracture 3 with three screws 4, 4', 4''.

The screws have been installed in the middle of the holes of the multilayer plate so that every screw acts as a so-called neutral guide.

After this one relatively large hole 10 (Figure 5b) is drilled through the multilayer plate on the other side of the bone fracture. Through this hole another smaller eccentric hole 11 is drilled so that the middle point of this smaller drill hole is further away from the fracture than the middle point of the larger drill hole (Figure 5b). This situation is examined schematically in the enlarged Figure 5c. After this the surgeon drives into the drill hole 11 a screw 4 equipped with a conical head, as is shown schematically in Figure 5d from above. The screw 4 creates the compression effect (the compression of fractured bone parts against each other). The compression effect can be increased by using also another compression screw. Additionally one can generally use at least one neutral screw also on this other side of the fracture.

The compression effect can also be caused by designing the holes of the multilayer plate eccentric by other ways and/or by using suitable auxiliary devices which cause compression. Different kinds of compression techniques are well-known in surgical techniques. They have been introduced e.g. in the book F. Séquin and R. Texhammar, "AO/ASIF Instrumentation", Springer, Berlin, 1981.

The single plates of the multilayer plate of this invention can be manufactured of biostable and/or absorbable polymer, polymer mixture, copolymer or polymer composite. For example following absorbable polymers given in Table 1 can be used for manufacturing of the devices of this invention:

Table 1. Absorbable polymers

Polyglycolide (PGA)

Copolymers of glycolide:
Glycolide/L-lactide copolymers (PGA/PLLA)
Glycolide/trimethylene carbonate copolymers (PGA/TMC)

Polylactides (PLA)

Stereocopolymers of PLA:
Poly-L-lactide (PLLA)
Poly-DL-lactide (PDLLA)
L-lactide/DL-lactide copolymers

Copolymers of PLA:
Lactide/tetramethylglycolide copolymers
Lactide/trimethylene carbonate copolymers
Lactide/δ-valerolactone copolymer
Lactide/ε-caprolactone copolymer
Polydepsipeptides
PLA/polyethylene oxide copolymers
Unsymmetrically 3,6-substituted poly-1,4-dioxane-2,5-diones

Poly-β-hydroxybutyrate (PHBA)
PHBA/β-hydroxyvalerate copolymers (PHBA/HVA)

Poly-β-hydroxypropionate (PHPA)
Poly-p-dioxanone (PDS)
Poly-δ-valerolactone
Poly-ε-caprolactone
Methylmethacrylate-N-vinyl pyrrolidine copolymers
Polyesteramides
Polyesters of oxalic acid
Polydihydropyrans
Polyalkyl-2-cyanoacrylates
Polyurethanes (PU)
Polyvinylalcohol (PVA)
Polypeptides
Poly-β-malic acid (PMLA)
Poly-β-alkanoic acids
Polyvinylalcohol (PVA)
Polyethyleneoxide (PEO)
Chitine polymers

Reference: S. Vainionpää, P. Rokkanen and P. Törmälä, Progr. Polym. Sci., in press.

It is self-evident that also other absorbable polymers than those given in Table 1 can be applied in manufacturing the devices or their parts of this invention. E.g. the following publications give absorbable (biodegradable) polymers which can be applied in this connection: U.S. Pat. No. 4 700 704 (Jamiolkows and Shalaby), U.S. Pat. No. 4 653 497 (Bezwada, Shalaby and Newman), U.S. Pat. No. 4 649 921 (Koelmel, Jamilkows and Bezwada), U.S. Pat. No. 4 559 945 (Koelmel and Shalaby), U.S. Pat. No. 4 532 928

EP 0 449 867 B1

(Bezwada, Shalaby and Jamiolkows), U.S. Pat. No. 4 605 730 (Shalaby and Jamiolkows), U.S. Pat. No. 4 441 496 (Shalaby and Koelmel), U.S. Pat. No. 4 435 590 (Shalaby and Jamiolkows).

The multilayer plates of this invention, which are manufactured of polymer, polymer mixture or copolymer, are of their structure at best reinforced composites.

Absorbable composites, like self-reinforced absorbable composites, are specially advantageous raw-materials for manufacturing the multilayer plates of this invention. Such self-reinforced composites have been described in publications U.S. Pat. No. 4 743 257 (Törmälä et al.) and WO88/05312 (Törmälä et al.). Also other types of absorbable composites can be applied in manufacturing of the multilayer plate of this invention. Accordingly multilayer plates can be manufactured by reinforcing absorbable material with fibers, film fibers, filaments, etc. which have been manufactured of absorbable polymer, copolymer or polymer mixture. The reinforcing can be carried out also with structures which have been constructed of above reinforcing elements like with braids, threads, bands, non-vowen stuctures, fabrics, knits etc. The reinforcing with the above reinforcing elements is made by combining the reinforcing elements and the absorbable polymer matrix with each other applying typical polymer technological methods. The reinforcing elements like absorbable fibers can be manufactured e.g. of several of the abosorbable polymers given in Table 1 and in the above text. The fibers can also be biodegradable ceramic fibers, like e.g. calcium phosphate fibers (see e.g. S. Vainionpää et al., progr. Polym. Sci., in press).

The multilayer plates of the invention comprising absorbable organic and/or unorganic absorbable fibers or structures constaucted of them and absorbable matrix, can be manufactured with various methods of plastics technology by winding the reinforcing structures at least partially to each other with absorbable polymer, copolymer or polymer mixture (matrix material) in such conditions, where matrix and reinforcing structures form homogeneous enough composite. Such structures are formes usually when the matrix is in liquid or melted state. Suitable methods to bind the reinforcing fibers etc. and matrix to each other and to form the composite material to semifinished products and/or implants are e.g. injection molding, extrusion, pultrusion, filament winding, compression winding and other plastics technological processing methods. The multilayer plate of the invention can also be manufactured of many biostable polymers, like polyethylene, polypropylene, polyasetal, polyamides, polyesters, polysulphone, polyetheretherketone, thermoplastic liquid crystalline polymers and other polymers.

Absorbable and/or biostable multilayer plate materials can also be reinforced with biostable fibers like with carbon fibers, ceramic fibers (e.g. $Al_2O_3$), polyester fibers etc. It is natural that the devices of the invention can additionally include different kind of additives or auxiliary chemicals to facilitate the processing of the material (e.g. stabilizators, antioxidants or plasticizers) or to change its properties (e.g. plasticizers or powder-like ceramic materials or biostable fibers like carbon fibers) or facilitate its handling (e.g. colours).

According to an advantageous embodiment the devices of the invention include some bioactive material or materials, like antibiotic, chemotherapeutic, chemicals accelerating the healing of the wound, growth hormones, anticoagulants (like heparine) etc. This kind of bioactive devices are especially advantageous in clinical use, because they have in addition to the mechanical effect also biochemical, chemotherapeutic, drug effect etc. in living tissues.

The fixation device of the invention has several advantages in comparison the known plate-like devices. The polymeric or polymer composite plate do not have the adverse corrosion effects of metal plates and the elastic modulus of the polymer or polymer composite plate can be regulated suitably in such a way that the porosity of the bone does not increase and the healing of the fracture occurs with the callus mechanism. A special advantage is that the drill holes of the devices of the invention can be done just in the operation room by drilling. In this way the drill holes can be tailormade into the plate in the best way for the treatment of the patient. A significant advantage of the multilayer plate of the invention in comparison to the known polymeric and composite plates is the fact that the multilayer plate of the invention can be bended easily without heating, which facilitates and simplifies the operation and gives an exact operational result.

The multilayer plate is also tougher than one thick polymer plate. The thick absorbable polymer plates have a tendency of breaking (see e.g. J. Eitenmüller et al. Abstracts of Eur. Congr. Biomater., Bologna, Italia, 1986, p. 94). Example 1 of this invention shows that the multilayer plates did not break in animal test. A evident reason to this is that the thin plates, about which the multilayer plate is constructed, are tougher than the thick plates. On the other hand, if a fracture nucleates in one plate of the multilayer plate, it does not necessarily lead to catastrophal fracture of the whole device as easily as a nucleated fracture in a thick homogeneous plate.

The multilayer plate of the invention can be applied in fixation of different kinds of bone fractures, osteotomies and arthrodeses.

6

It can also be applied for fixation of different kinds of prostheses like synthetic ligaments or ligament transplants or for fixation of other tissues into or on bone tissue by locating at least part of the ligament, tissue etc. between the plates of the multilayer plate and/or between the bone tissue and the multilayer plate.

One special advantage of the multilayer plate of the invention in comparison to the known plates is that surgeon can manufacture the multilayer in operation room taking into account the size of the bone and/or the fracture of the patient. The multilayer plate can be easily constructed in the operation room by cutting suitable parts from bigger polymer- or composite plate e.g. with scissors, stamping device, cutter, saw, hot wire, laser cutter etc. other cutting device desirable plates and by combining them to a multilayer plate (plate bundle) of the invention. By using such tailormade multilayer plates it is possible to optimize the fixation effect and at the same time to minimize the foreign material amount which is impanted into the tissues of the patient.

Single plates of the multilayer plate of the invention can be fixed to each other with a binding material layer which is very elastic or easily deformable with plastic deformation. This makes the treatment of the multilayer plate in operation room easier when the single plates have been bound to each other. When this kind of multilayer plate is bent on the bone surface according to its surface curvature, the binding material layer deforms (elastic or plastic deformation) which facilitates the gliding of the single plates to each other giving at last for the multilayer plate the desired shape. Suitable binding materials to the above multilayer plate are flexible and soft polymers, e.g. -caprolactone and its copolymers (e.g. with lactide), ethylene oxide-lactide copolymers, amorphous lactides like poly-DL-lactide. It is natural that also other types of elastic or plastic polymers or waxy oligomers etc. can be applied as binding material.

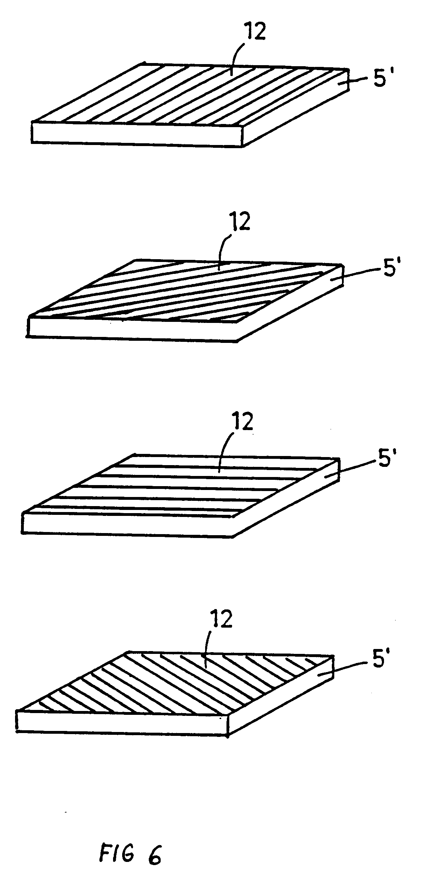

When the multilayer plate of the invention is manufactured of oriented and/reinforced plates, the different plates can be manufactured of materials oriented and/or reinforced in different directions. In this case a multilayer plate structure, which is strong in different directions can be constructed. The structure of such a multilayer plate has been schematically in Figure 6 where the orientation of molecular chains and/or the orientation of reinforcement elements (like reinforcing fibers) in different single plates 5' of a multilayer plate has described with black lines 12.

The invention and its function has been illustrated in details with the following examples.

EXAMPLE 1.

The fixation of the osteotomy of sheep mandibula with polylactide plates.

Polylactide plates with the thickness 2.0 mm and other dimensions 50 x 50 mm were manufactured by injection molding from poly-L-lactide (Mw 250.000, manufacturer CCA biochem, Holland). The plates were drawn at 90°C to the final thickness of 0.5 mm. The plates self-reinforced during the drawing. The drawn plate was cut in the drawing direction to 60 mm long and 12 broad plates. Four such plates were stacked on each other to form a multilayer plate. The plates were sterilized by immersing them for 5 minutes in ethanol/water (75 w/w-% of ethanol) mixture.

A sheep was anesthetized. The skin area on the right side of mandibular was shaved and washed with Neo-Amisept. The skin was opened and the periosteum of mandibula was revealed. The diastema area of mandibula was revealed and the mandibula was osteotomised in this area. The cut part of mandibula was fixed temporarily from the upper part of the bone with a fracture clamp. The bone tissue was dissected free of soft tissues 3 cm to both sides of the osteotomy. A 0.5 mm thick, 12 mm broad and 60 mm long self-reinforced polylactide plate was located on the dissected area so that the middle point of the plate was above the osteotomy. Three identical plates were stacked on this first plate by hand and the plates were bent according to the form of the bone. The multilayer plate comprising four single plates was fixed with plate clamps to the both pieces of mandibula so that a good contact of the osteotomized fragments remained. A 2.5 mm diameter drill hole was drilled through the multilayer plate to the end of the plate near the condyle. Through this drill hole a 2 mm diameter drill hole was drilled into the bone through both cortexes. This drill hole was drilled in the middle of the bigger hole in the multilayer plate (neutral guide). The length of the drill hole in bone was measured and it was threaded. A 2.5 mm diameter titanium screw was driven into the bone so that the multilayer plate was now fixed with one screw on the mandibula. Altogether three drill holes were done into the condylar part of the multilayer plate and into every drill hole one screw with the above given neutral technique was fixed. On the other side of the osteotomy a 3.2 mm diameter drill hole was drilled through the multilayer plate near the osteotomy and an eccentric drill hole (2.0 mm diameter) was drilled with an eccentric guide into the bone through both cortexes in such a way that the middle point of the drill in the bone was 0.8 mm further from the osteotomy line than the middle point of the drill hole in the multilayer plate. A titanium screw was driven into the drill hole in the bone so that the screw created 0.3 mm compression effect into the plate; first the screw was not driven totally to the end, because also the following (second screw) was fixed with the same technics. Accordingly two eccentric screws were applied to create the compression effect (it should have been possible also to use only one eccentric screw). Also this second part of the multilayer plate (on the distant side of the osteotomy in relation to the condyle) was fixed altogether with three screws so that one screw was fixed with the neutral guide principal. With this technique it was possible to fix the multilayer plate on the bone surface so that the multilayer plate followed the form of the bone and the screws in the drill holes of the multilayer plate prevent it from straightening. So the multilayer plate fixed the osteotomy steadily with the slight compression. Subcutaneous tissue and the skin were closed with absorbable suture (Dexon). After the operation the sheep got for one week liquid-like food. Altogether 14 sheeps were operated with the above technics. X-ray figures were taken from all osteotomies after three weeks. Three animals were sacrificed 6, 12 and 24 weeks after operation and the mandibulas were reprepared carefully, x-ray figures were taken from mandibula and the bone was cast into methylmethacrylate. Histological observations showed that ossifying of the osteotomies was in progress after six weeks and after 12 weeks all the osteotomies were ossified. After one and two years' follow-up x-ray figures showed normal ossifying. Histological study from one mandibula afetr two years' follow-up time showed that the biodegradation of polylactide plates had started.

A comparative study was done with one sheep, for which the osteotomy was fixed with one injection molded 2 mm thick PLLA plate (breadth 12 mm, length 60 mm, fixation with 6 titanium screws). Six weeks after operation the plate had broken around two fixation screws and the osteotomy did not ossify, but a connective tissue bridge was formed.

EXAMPLE 2.

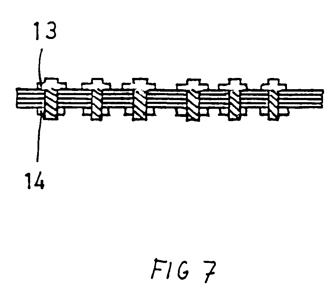

The following polymers were applied to manufacture by injection molding 2 x 60 x 80 big plates: poly-L-lactide, polyglycolide, poly-$\beta$-hydroxybutyrate, glycolide/lactide copolymer (Polyglactine 910), HD-polyethylene, polypropylene, HD-polyethylene + polypropylene mixture (mole ratio 1:1), polysulphone, polyetheretherketone. The plates were to the thickness of 0.5 mm at temperatures $T = T_g + (10\text{-}60\,°C)$, where $T_g$ = the glass transition temperature of the polymer. The drawn plates were cut in the drawing direction to 70 mm long and 10 mm broad plates. All such cut plates were stacked on each other to form a multilayer plate. Six drill holes with a distance of 10 mm between single drill holes were drilled through the multilayer plate with a 2 mm diameter drill. Threaded bolts 13 with the diameter of 2.2 mm were driven into the holes and the multilayer plate comprising of four single plates were tightened to tight bundles by driving nuts 14 (see Figure 7) to the other ends of the bolts and by tightening the nuts. The bending strengths of the multilayer plates fixed with bolt were compared with the bending of corresponding single plates with dimensions 2 x 10 x 70 mm which were perforated and bolted in an analogous way. The bending strengths of the multilayer plates were 1.5 - 4 times higher than the bending strengths of the injection molded plates.

**Claims**

1. A plate for fixation of a bone fracture, osteotomy, arthrodesis or corresponding or for fixation of a ligament, tendon or connective tissue on the bone, said plate being intended to be fixed on the bone at least with one fixation device (4) like screw, rod, clamp or corresponding, wherein the plate comprises at least two essentially superimposed plates, so as to provide a multilayer plate construction (5), in which the plates have been manufactured of a polymer copolymer, polymer alloy or polymer composite, **characterized** in that the individual plates of said multilayer plate construction (5) are flexible so as to provide a change of form or said multilayer plate construction to substantially assume the shape of the bone surface in the operation conditions by means of an external force such as by hand and/or by bending instrument directed to said multilayer plate construction, whereby each individual plate assumes the position of its own with respect to other individual plates by differential motion along the surfaces of coinciding plates.

2. A plate according to claim 1, **characterized** in that the individual plates have been joined to each other by means of an intermediate layer between the surfaces of the coinciding plates to allow the differential motion of the individual plates of said multilayer construction (5) e.g. the gliding of the plates in respect to each other in operation conditions.

3. A plate according to claims 1 and/or 2, **characterized** in that the individual plates are locked with respect to each other to accomplish a substantially stiff multilayer plate construction (5) by means of

EP 0 449 867 B1

frictional force between the individual plates, said frictional force being accomplished by the effects of said at least one fixation device (4) during the fixation of said multilayer plate construction (5) on the bone surface.

4. A plate according to any claim of claims 1-3, **characterized** in that said multilayer plate construction (5) is provided with at least two holes (8) through each individual plate of said multilayer plate construction (5), the diametral measure of said holes being at least in one diametral direction greater that the greatest diametral measure of the part of the fixation device (4) to be positioned through said hole thus providing a certain dislocation between the holes in the individual plates with respect to each other without the risk that said hole (8) through said multilayer plate construction (5) becoming unable to receive said fixation device (4).

5. A plate according to any claim of claims 1-4, **characterized** in that said multilayer plate construction (5) is provided with at least two oblong holes (8) through each individual plate of said multilayer plate construction (5) the direction of the oblongness being arranged preferably substantially in a direction allowing the maximum differential motion between said individual plates.

6. A plate according to any claim of claims 1-5, **characterized** in that at least part of said individual plates of said multilayer plate construction (5) has been manufactured of biostable polymer.

7. A plate according to any claim of claims 1-6, **characterized** in that at least part of said individual plates of said multilayer plate construction (5) have been manufactured of absorbable polymer.

8. A plate according to any claim of claims 1-6, **characterized** in that at least part of said individual plates of said multilayer plate construction (5) have been reinforced with biostable reinforcing elements, like fibers or structures which have been constructed of fibers.

9. A plate according to any claim of claims 1-8, **characterized** in that at least part of said individual plates of said multilayer plate construction (5) have been reinforced with absorbable reinforcement elements, like with fibers or structures constructed of fibers.

10. A plate according to any claim of claims 1-9, **characterized** in that at least part of said individual plates of said multilayer plate construction (5) are self-reinforced.

11. A plate according to any claim of claims 8-10, **characterized** in that said reinforcement elements and/or the molecular chains in the separate individual plates of said multilayer plate construction (5) are orientated in different directions.

**Patentansprüche**

1. Platte zur Befestigung einer Knochenfraktur, Osteotomie, Arthrodese oder entsprechendem oder zur Befestigung eines Ligaments, Sehne oder Bindegewebe an dem Knochen mit wenigstens einer Befestigungsvorrichtung (4), wie einer Schraube, einem Stab, einer Klammer oder entsprechendem, wobei die Platte wenigstens zwei im wesentlichen übereinander angeordnete Platten umfaßt, um eine Mehrschichtplattenkonstruktion (5) zu bilden, in welcher die Platten aus einem Polymeren, Copolymeren, polymeren Legierung oder einer polymeren Zusammensetzung hergestellt sind, dadurch gekennzeichnet, daß die Einzelplatten der Mehrschichtplattenkonstruktion (5) flexibel sind, um eine Änderung der Form der besagten Mehrschichtplattenkonstruktion zu ergeben, um im wesentlchen die Form der Knochenoberfläche unter Operationsbedingungen bei einer äußeren Kraft, wie durch Hand und/oder durch ein Biegeinstrument, das auf die besagte Mehrschichtplattenkonstruktion gerichtet ist, anzunehmen, wodurch jede Einzelplatte ihre eigene Position gegenüber den anderen Einzelplatten durch Differentialbewegung entlang der Oberflächen koinzidierender Platten einnimmt.

2. Platte nach Anspruch 1, dadurch gekennzeichnet, daß die Einzelplatten miteinander mit einer Zwischenschicht zwischen den Oberflächen koinzidierender Platten verbunden worden sind, um eine Differentialbewegung der Einzelplatten der besagten Mehrschichtkonstruktion (5), z. B. des gegenseitigen Gleitens der Platten unter Operationsbedingungen zu ermöglichen.

9

**3.** Platte nach Anspruch 1 und/oder 2, dadurch gekennzeichnet, daß die Einzelplatten gegenseitig blockiert sind, um eine im wesentlichen steife Mehrschichtplattenkonstruktion (5) durch Reibungskraft zwischen den Einzelplatten zu bilden, wobei die Reibungskraft durch die Wirkungen der besagten wenigstens einen Befestigungsvorrichtung (4) während der Befestigung der besagten Mehrschichtplattenkonstruktion (5) auf der Knochenoberfläche gebildet wird.

**4.** Platte nach irgendeinem Anspruch der Ansprüche 1 - 3, dadurch gekennzeichnet, daß die besagte Mehrschichtplattenkonstruktion (5) mit wenigstens zwei Löchern (8) durch jede Einzelplatte der besagten Mehrschichtplattenkonstruktion (5) versehen ist; wobei die Durchmesser-Abmessung der besagten Löcher wenigstens in einer Durchmesser-Richtung größer ist als die größte Durchmesser-Abmessung des Teils der Befestigungsvorrichtung (4), das durch das Loch angeordnet wird, um damit eine gewisse Verschiebung zwischen den Löchern in den Einzelplatten gegenüber einander vorzusehen ohne das Risiko, daß das besagte Lich (8) durch die Mehrschichtplattenkonstruktion (5) nicht in der Lage ist, die besagte Befestigungsvorrichtung (4) aufzunehmen.

**5.** Platte nach irgendeinem Anspruch der Ansprüche 1 - 4, dadurch gekennzeichnet, daß die besagte Mehrschichtplattenkonstruktion (5) mit wenigstens zwei Langlöchern (8) durch jede Einzelplatte der besagten Mehrschichtplattenkonstruktion (5) versehen ist, wobei die Richtung der Länglichkeit vorzugsweise im wesentlichen in einer Richtung angeordnet ist, um die maximale Differentialbewegung zwischen den besagten Einzelplatten zu ermöglichen.

**6.** Platte nach irgendeinem Anspruch der Ansprüche 1 - 5, dadurch gekennzeichnet, daß wenigstens ein Teil der besagten Einzelplatten der besagten Mehrschichtplattenkonstruktion (5) aus einem biostabilen Polymeren hergestellt worden ist.

**7.** Platte nach irgendeinem Anspruch der Ansprüche 1 - 6, dadurch gekennzeichnet, daß wenigstens ein Teil der besagten Einzelplatten der besagten Mehrschichtplattenkonstruktion (5) aus einem absorbierbaren Polymeren hergestellt worden sind.

**8.** Platte nach irgendeinem Anspruch der Ansprüche 1 - 7, dadurch gekennzeichnet, daß wenigstens ein Teil der besagten Einzelplatten der besagten Mehrschichtplattenkonstruktion (5) mit biostabilen Verstärkungselementen, wie Fasern oder Strukturen, welche mit Fasern aufgebaut worden sind, verstärkt ist.

**9.** Platte nach irgendeinem Anspruch der Ansprüche 1 - 8, dadurch gekennzeichnet, daß wenigstens ein Teil der besagten Einzelplatten der besagten Mehrschichtplattenkonstruktion (5) durch absorbierbare Verstärkungselemente, wie Fasern oder Strukturen, die aus Fasern aufgebaut sind, verstärkt ist.

**10.** Platte nach irgendeinem Anspruch der Ansprüche 1 - 9, dadurch gekennzeichnet, daß wenigstens ein Teil der besagten Einzelplatten der besagten Mehrschichtplattenkonstruktion (5) selbst verstärkt ist.

**11.** Platte nach irgendeinem Anspruch der Ansprüche 8 - 10, dadurch gekennzeichnet, daß die Verstärkungselemente und/oder die Molekülketten in separaten Einzelplatten der besagten Mehrschichtplattenkonstruktion (5) in unterschiedliche Richtungen orientiert sind.

**Revendications**

**1.** Plaque pour la fixation d'une fracture osseuse, d'une ostéotomie, d'une arthrodèse ou d'une opération correspondante, ou pour la fixation d'un ligament, d'un tendon ou d'un tissu conjonctif sur l'os, ladite plaque étant destinée à être fixée sur l'os au moins à l'aide d'un dispositif de fixation (4) comme par exemple une vis, une tige, une pince ou un dispositif correspondant, ladite plaque comprenant au moins deux plaques essentiellement superposées, de manière à réaliser une construction de plaque multicouche (5), dans laquelle les plaques ont été fabriquées en un polymère, un copolymère, un alliage de polymère ou un composite de polymère, caractérisée en ce que les plaques individuelles de ladite construction de plaque multicouche (5) sont flexibles de manière à assurer un changement de forme pour ladite construction de plaque multicouche afin d'adopter sensiblement la forme de la surface de l'os dans les conditions opératoires au moyen d'une force externe telle qu'appliquée à la main et/ou à l'aide d'un instrument de coudage sur ladite construction de plaque multicouche, grâce à quoi chaque plaque individuelle adopte sa propre position par rapport aux autres plaques individuelles par déplacement différentiel le long des surfaces de plaques superposées.

2. Plaque selon la revendication 1, caractérisée en ce que les plaques individuelles ont été reliées les unes aux autres au moyen d'une couche intermédiaire entre les surfaces des plaques superposées afin de permettre le mouvement différentiel des plaques individuelles de ladite construction multicouche (5) par exemple le coulissement des plaques les unes par rapport aux autres dans des conditions opératoires.

3. Plaque selon l'une et/ou l'autre des revendications 1 et 2, caractérisée en ce que les plaques individuelles sont bloquées les unes par rapport aux autres afin de réaliser une construction de plaque multicouche (5) sensiblement rigide, au moyen des forces de friction entre les plaques individuelles, lesdites forces de friction étant accomplies par les effets dudit au moins un dispositif de fixation (4) pendant la fixation de ladite construction (5) de plaque multicouche sur la surface de l'os.

4. Plaque selon l'une quelconque des revendications 1 à 3, caractérisée en ce que ladite construction (5) de plaque multicouche est pourvue d'au moins deux trous (8) à travers chaque plaque individuelle de ladite construction (5) de plaque multicouche, la dimension en diamètre desdits trous étant, au moins dans une direction diamétrale, supérieure à la plus grande taille diamétrale de la partie du dispositif de fixation (4) qui doit être placé à travers ledit trou, assurant ainsi une certaine délocalisation entre les trous des plaques individuelles les uns par rapport aux autres sans risquer que ledit trou (8) à travers ladite construction (5) de plaque multicouche ne devienne incapable de recevoir ledit dispositif de fixation (4).

5. Plaque selon l'une quelconque des revendications 1 à 4, caractérisée en ce que ladite construction (5) de plaque multicouche est pourvue d'au moins deux trous oblongs (8) à travers chaque plaque individuelle de ladite construction (5) de plaque multicouche, la direction de l'allongement desdits trous étant de préférence agencée sensiblement dans une direction qui permet le déplacement différentiel maximum entre lesdites plaques individuelles.

6. Plaque selon l'une quelconque des revendications 1 à 5, caractérisée en ce qu'au moins une partie desdites plaques individuelles de ladite construction de plaque multicouche (5) a été produite en polymère stable sur le plan biologique.

7. Plaque selon l'une quelconque des revendications 1 à 6, caractérisée en ce qu'au moins une partie desdites plaques individuelles de ladite construction (5) de plaque multicouche ont été produites en polymère absorbable.

8. Plaque selon l'une quelconque des revendications 1 à 6, caractérisée en ce qu'au moins une partie desdites plaques individuelles de ladite construction (5) de plaque multicouche ont été renforcées à l'aide d'éléments de renfort stables sur le plan biologique, tels que des fibres ou des structures qui ont été construites avec des fibres.

9. Plaque selon l'une quelconque des revendications 1 à 8, caractérisée en ce qu'au moins une partie desdites plaques individuelles de ladite construction (5) de plaque multicouche ont été renforcées avec des éléments de renfort absorbables, tels que des fibres ou des structures construites avec des fibres.

10. Plaque selon l'une quelconque des revendications 1 à 9, caractérisée en ce qu'au moins une partie desdites plaques individuelles de ladite construction (5) de plaque multicouche sont auto-renforcées.

11. Plaque selon l'une quelconque des revendications 8 à 10, caractérisée en ce que lesdits éléments de renfort et/ou les chaînes moléculaires dans les plaques individuelles séparées de ladite construction (5) de plaque multicouche sont orientés dans des directions différentes.

FIG 1

FIG 2

FIG 3

(a)

(b)

FIG 4

FIG 5

FIG 6

FIG 7